# EUROPÄISCHE PATENTANMELDUNG

(11) **EP 0 699 441 A1**
(43) Veröffentlichungstag der Anmeldung: **06.03.1996**
(21) Anmeldenummer: 95109903.5
(22) Anmeldetag: 25.06.1995
(51) Int. Cl.: A61K 35/12, A61K 35/14

(54) **Mittel zur Behandlung des atopischen Ekzems und Verfahren zur Herstellung desselben**

(30) Priorität: 09.07.1994 DE 4424205
(71) Anmelder: Plewig, Gerd, Prof. Dr. med., D-80337 München (DE); Prinz, Bettina, Dr., D-80337 München (DE)
(72) Erfinder: Plewig, Gerd, Prof. Dr. med., D-80337 München (DE); Prinz, Bettina, Dr., D-80337 München (DE)
(74) Vertreter: Werner, Hans-Karsten, Dr.

(57) **Zusammenfassung**

Das Mittel zur Behandlung des atopischen Ekzems ist erhältlich durch orale Applikation eines Psoralens, Entnahme von Blut, Auftrennung des entnommenen Blutes in Plasma und eine leukozytenreiche Fraktion und Behandlung der leukozytenreichen Fraktion mit UV-A-Strahlung. Es ist besonders geeignet zur Behandlung von schweren Fällen des atopischen Ekzems.

## Beschreibung

Gegenstand der vorliegenden Erfindung ist ein Mittel zur Behandlung des atopischen Ekzems sowie eines Verfahrens zur Herstellung dieses Mittels.

Das atopische Ekzem ist eine häufige auf mehreren Faktoren beruhende chronische und oft wiederkehrende entzündliche Hautkrankheit. Es ist gekennzeichnet durch kutane Erytheme, Verhärtung und schweren Pruritus. Die klinischen Manifestationen der Krankheit sind Auswirkungen des bestimmten Typs von Immunreaktion, welches eine exzessive Überproduktion von IgE-Antikörpern umfaßt, welches sowohl die überschießende Aktivierung von bestimmten T-Zellen Untergruppen in der Haut sowie das Auftreten eines bestimmten Musters von inflammatorischen Mediatoren umfaßt. Dies wird als unabdingbare Voraussetzung der Manifestation dieser Krankheit angesehen.

Die Erfindung hat sich die Aufgabe gestellt, das atopische Ekzem, insbesondere das schwere atopische Ekzem, zu behandeln und dazu ein geeignetes Mittel zur Verfügung zu stellen.

Diese Aufgabe konnte jetzt überraschend dadurch gelöst werden, daß dem Patienten zunächst oral ein Psoralen appliziert wird, ihm dann Blut entnommen wird, dieses in Plasma und eine leukozytenreiche Fraktion aufgetrennt und die leukozytenreiche Fraktion mit UV-A-Strahlung behandelt wird.

Diese so behandelte Fraktion des Blutes ist das neue Mittel, welches dem Patienten injiziert wird und die gewünschten Wirkstoffe zur Behandlung der schweren Fälle des atopischen Ekzems enthält.

Als oral zu applizierendes Psoralen kommen prinzipiell alle bekannten Substanzen dieser Stoffklasse in Frage. Bevorzugt sind 8-MOP (8-Methoxypsoralen), 5-MOP (5-Methoxypsoralen) sowie das synthetische Psoralen TMP (4,5',8-Trimethylpsoralen). Prinzipiell in Frage kommen aber auch andere Psoralene und anguläre Furocumarine (Angelicine) wie 7-Methylpyrodopsoralen (7-MPP) und 4,6,4'-Trimethylangelicin.

Die Entnahme von Blut, Auftrennung in Plasma und eine leukozytenreiche Fraktion und Behandlung dieser Fraktion mit UV-A-Strahlung in Gegenwart einer zugesetzten sterilen Lösung eines Psoralens ist in der EP-A-0 392 429 beschrieben und dient zur Behandlung von immunologisch verursachten Erkrankungen wie T-Zellen-Lymphom, Sklerodermie, Lupus erythematodes und Pemphigus vulgaris.

Diese sterilen Lösungen enthalten außer dem Psoralen Ethylalkohol und Propylenglycol. Sie sind arzneimittelrechtlich noch nicht zugelassen, so daß auch nicht bekannt ist, welche Nebenwirkungen auftreten können. Orale Applikationsformen der Psoralene sind hingegen bekannt und zugelassen, beispielsweise das 8-MOP unter der Bezeichnung Meladinine®.

Das erfindungsgemäße Mittel kann erfolgreich eingesetzt werden bei Patienten mit schwerem atopischen Ekzem, welches sich anderen üblichen Behandlungsmethoden widersetzt hat, insbesondere bei Patienten, die resistent waren gegen die üblichen etablierten therapeutischen Methoden gegen das atopische Ekzem. Es wurde eine deutliche Abnahme der kutanen inflammatorischen Aktivität bereits am Ende des zweiten Photophoresezyklus beobachtet. Bei einigen Patienten sind die Hautläsionen nach fünf Behandlungszyklen verschwunden. Bei anderen Patienten wurde eine dauerhafte und erhebliche Verbesserung von Pruritus und Erythem beobachtet. Die klinische Remission war unter Erhaltungstherapie für längere Zeit zwischen den Photophoresezyklen gegeben. Die therapeutische Wirksamkeit ist parallel zu einer gemessenen Reduktion des IgE-Serum-Spiegels, während die Serumspiegel von IgG, IgM und IgA sowie das Gesamtprofil der zirkulierenden Lymphozyten im wesentlichen unverändert blieben. Es wurden auch keine Anzeichen von Immunsupression oder anderen schweren Nebenwirkungen beobachtet.

Die Intervalle der Behandlung betrugen im allgemeinen vier Wochen. Es ist jedoch davon auszugehen, daß in schwereren Fällen auch kürzere Behandlungsintervalle zur Anwendung kommen können, während leichtere Fälle mit längeren Behandlungszyklen auskommen können.

Die Behandlung der leukozytenreichen Fraktion mit UV-A-Strahlung wurde durchgeführt mit einem UV-A-System II, wie es von Therakos, Johnson & Johnson Co., USA kommerziell angeboten wird. Die Patienten erhielten etwa zwei Stunden vor der Behandlung 0,6 mg/kg 8-MOP (Meladinine®, Basotherm Deutschland). Das Blut der Patienten wurde gesammelt mittels eines Katheders und kontinuierlich zentrifugiert, wobei 240 ml mit Leukozyten angereichertes Blut und 300 ml Plasma entstanden. Das Plasma wurde dem Patienten jeweils direkt wieder zugeführt, während die mit Leukozyten angereicherte Fraktion der UV-Bestrahlung ausgesetzt wurde mit einer Wellenlänge von 334 bis 336 nm. Die der Probe zugeführte Photoenergie betrug 2 J/cm² für die DNA der weißen Blutpartikel. Danach wurde auch diese behandelte, mit Leukozyten angereicherte Fraktion den Patienten reinfundiert. Die anfänglichen Intervalle von vier Wochen wurden nach einiger Zeit auf sechs Wochenabstände verlängert. Die Patienten können während der Therapie gewünschtenfalls topisch mit indifferenten Hautpflegemitteln oder schwach wirksamen Steroiden behandelt werden. Diese Steroide waren aber in allen bisher behandelten Fällen für sich alleine nicht oder nicht mehr wirksam.

Die begleitenden Untersuchungen der sonstigen Parameter zeigten, daß es zu keinen Veränderungen kamen außer einer deutlichen Absenkung der IgE-Serumspiegels.

Gegenstand der vorliegenden Erfindung sind somit das Mittel zur Behandlung des atopischen Ekzems sowie das Verfahren zur Herstellung dieses Mittels gemäß den Patentansprüchen 1 bis 4.

## Patentansprüche

1. Mittel zur Behandlung des atopischen Ekzems, erhältlich durch orale Applikation eines Psoralens, Entnahme von Blut, Auftrennung des entnommenen Blutes in Plasma und eine leukozytenreiche Fraktion und Behandlung der leukozytenreichen Fraktion mit UV-A-Strahlung.

2. Mittel gemäß Anspruch 1, dadurch gekennzeichnet, daß als oral appliziertes Psoralen eine oder mehrere Substanzen aus der Gruppe 8-MOP, 5-MOP und TMP verwendet wird.

3. Verfahren zur Herstellung eines Mittels zur Behandlung des atopischen Ekzems, dadurch gekennzeichnet, daß dem Patienten oral ein Psoralen appliziert und dann Blut entnommen wird, dieses in Plasma und eine leukozytenreiche Fraktion aufgetrennt und die leukozytenreiche Fraktion mit UV-A-Strahlung behandelt wird.

4. Verfahren gemäß Anspruch 3, dadurch gekennzeichnet, daß als oral appliziertes Psoralen eine oder mehrere Substanzen aus der Gruppe 8-MOP, 5-MOP und TMP verwendet wird.
